# FASCICULE DE BREVET EUROPEEN

(11) **EP 1 325 051 B1**
(45) Date de publication et mention de la délivrance du brevet: **20.08.2008**
(21) Numéro de dépôt: 01976398.6
(22) Date de dépôt: 11.10.2001
(51) Int. Cl.: C08G 18/22, C08G 18/71, C08G 18/28, C07C 271/06

(54) **UTILISATION COMME CATALYSEUR POUR LA FORMATION D'URETHANES, DE SELS D'ACIDE FLUORES ET DE METAL TRIVALENT, COMPOSITION EN CONTENANT ET PROCEDE**
VERWENDUNG VON SALZEN FLUORIERTER SÄUREN UND DREIWERTIGER METALLE ALS KATALYSATOREN ZUR URETHANBILDUNG, ZUSAMMENSETZUNG DIE DIESE BEINHALTET UND VERFAHREN
USE AS CATALYST FOR FORMING URETHANES, OF FLUORINATED AND TRIVALENT METAL ACID SALTS, COMPOSITION CONTAINING SAME AND METHODS USING SAME

(30) Priorité: 13.10.2000 FR 0013162; 13.10.2000 FR 0013161
(43) Date de publication de la demande: 09.07.2003
(73) Titulaire: RHODIA CHIMIE, 92512 Boulogne Billancourt Cedex (FR)
(72) Inventeur: BERNARD, Jean-Marie, F-69440 MORNANT (FR); JOUSSEAUME, Bernard, F-33400 TALENCE (FR); LAPORTE, Christian, F-31320 CASTANET-TOLOSAN (FR); TOUPANCE, Thierry, F-33400 TALENCE (FR)
(74) Mandataire: Ricalens, François
(86) Numéro de dépôt international: PCT/FR2001/003144
(87) Numéro de publication internationale: WO 2002/031014

(56) Documents cités:
- US-A- 4 018 744
- US-A- 4 386 033
- US-A- 5 206 412
- US-A- 5 430 122
- US-A- 5 527 266
- PATENT ABSTRACTS OF JAPAN vol. 011, no. 242 (C-438), 7 août 1987 (1987-08-07) & JP 62 048716 A (NIPPON SHEET GLASS CO LTD), 3 mars 1987 (1987-03-03)

## Description

La présente invention a pour objet de nouveaux catalyseurs de transcarbamatation. Elle concerne plus spécifiquement l'utilisation de nouveaux catalyseurs qui ne soient pas à base d'étain.

En raison de leur forte réactivité et de leur relative toxicité, les isocyanates sont souvent utilisés sous une forme dérivée ou sous une forme masquée. Cette forme dérivée ou cette forme masquée présente l'inconvénient correspondant à ses avantages, c'est-à-dire qu'elle est peu réactive, aussi nécessite-t-elle l'utilisation de catalyseurs pour que la réaction puisse avoir lieu à des températures acceptables par l'industrie.

Les isocyanates masqués sont utilisables dans toutes les applications des isocyanates, à savoir les peintures, les vernis et plus généralement les revêtements, les colles et adhésifs, ainsi que certains polymères de spécialité.

Les catalyseurs les plus utilisés sont les alcanoates de dialcoylétain dont le plus connu est le dilaurate de dibutylétain. Cependant, pour certaines applications, le dilaurate de dibutylétain présente une activité insuffisante, aussi est-on obligé de l'utiliser à de très fortes concentrations.

Ces catalyseurs de (trans)carbamatation permettent de réaliser des polyuréthanes, notamment des polyuréthanes aliphatiques.

Toutefois, les sels d'étain ont une réputation de toxicité qui nuit à leur usage, aussi cherche-t-on pour de nombreuses applications à les remplacer par des composés ne présentant pas ces inconvénients.

En outre, l'industrie est toujours à l'affût de composés ayant des activités catalytiques supérieures aux catalyseurs existants.

Le catalyseur le plus utilisé comme référence dans le domaine des polyuréthanes est le dilaurate de dibutylétain.

US 5 430 122 décrit un procédé de préparation de poly(éther-uréthanes), par réaction d'éthers cycliques avec des isocyanates organiques en présence de catalyseurs métalliques.

US 5 206 412 divulgue un procédé de préparation de précurseurs de diphénylméthanedi-isocyanates, notamment dicarbamates ou bis(dialkylurées) de diphénylméthane, à partir d'un méthylène bis(alkylcarbamate) en présence d'un catalyseur acide.

JP 62 048716 a pour objet un procédé mettant en oeuvre un sel de métal alcalin, un polyéthylèneglycol trifonctionnel et un dérivé de di-isocyanate aliphatique pour la préparation d'un électrolyte polymère solide.

Dans le document US 5 527 266 est décrit une composition de résine polyuréthane réticulable à l'humidité et qui comprend un prépolymère polyuréthane obtenu à partir d'un polyol et d'un isocynate, en présence d'un catalyseur de réticulation et d'une quantité limitée d'acide trifluorométhane sulfonique en tant que stabilisant.

US 4 386 033 traite d'un procédé de coupure thermique d'un ester d'acide carbamique en alcool et en isocyanate, lequel isocyanate pouvant servir comme produit de départ pour une réaction de transuréthanation conduisant à la formation d'un isocyanate de point d'ébullition moins élevé.

US 4 018 744 divulgue des compositions stables au stockage comprenant un poly(ester d'acide carbamique) organique, un poly(ester d'acide carboxylique) organique d'un monoalcool, un composé polyhydroxylé organique et un catalyseur de condensation-réarrangement de type organo-étain.

Un des buts de la présente invention est donc de fournir un catalyseur de carbamatation qui soit au moins aussi actif que le dilaurate de dibutylétain, mais qui ne soit pas à base d'étain.

Un autre but de la présente invention est de fournir un catalyseur qui soit utilisable pour la synthèse des polyuréthanes, notamment de nature aliphatique.

Un autre but de la présente invention est de fournir un catalyseur du type précédent qui permette de réaliser la synthèse de polyuréthane à partir de polyisocyanates masqués.

Un autre but de la présente invention est de fournir un catalyseur qui permette de réaliser une transcarbamatation conduisant à des polyuréthanes à partir de carbamates d'alcoyles légers ; le terme «alcoyle» correspondant à un alcool dont le point d'ébullition sous pression ordinaire est au plus égal à 150°C (2 chiffres significatifs), avantageusement au plus 100°C (2 chiffres significatifs).

Un autre but de la présente invention est de fournir un catalyseur du type précédent qui puisse être utilisé lorsque les uréthanes ou polyuréthanes servent de liant au revêtement (vernis ou peintures).

Ces buts, et d'autres qui apparaîtront par la suite, sont atteints au moyen de l'utilisation comme catalyseur de composés répondant à la formule générale (I) suivante :

MY _{3-q}[Z]_{q},

où :
• Z est le radical correspondant à l'anion d'un super acide dont la constante de Hammett est au moins égale à 13 et avantageusement supérieure à 13 ;
• M représente un métal trivalent, de préférence connu pour former des acides de Lewis ;
• Y est un anion ou une fonction anionique monovalente et ;
• q est un entier choisi avantageusement entre 1 et 3, y compris les bornes.

Avantageusement Z est de formule (II) :

(Rₓ)-SO₂-ζ

où :
• Rₓ est un fluor, ou avantageusement un radical, dont le carbone porteur de la fonction sulfonique est perhalogéné, et
• ζ est un oxygène ou un atome de la colonne V, de préférence l'azote, monosubstitué par un radical électroattracteur GEA, avantageusement par un radical de formule (R'ₓ)-SO₂-, où R'ₓ est choisi parmi les mêmes radicaux que Rₓ.

GEA peut être choisi parmi les mêmes familles que Rₓ, avec les mêmes préférences que ce dernier. Il en va de même pour R'ₓ. Rₓ, R'ₓ et GEA ; ces radicaux peuvent être identiques ou différents.

ζ est le plus souvent oxygène pour donner une formule (l') :

MY_{3-q}[(Rₓ)-SO₂₋O-]_{q},

où :
• M représente un métal trivalent, de préférence connu, pour former des acides de Lewis ;
• Y est un anion ou une fonction anionique monovalente et ;
• Rₓ est un fluor, ou avantageusement un radical, dont le carbone porteur de la fonction sulfonique est perhalogéné, et
• q est un entier choisi avantageusement entre 1 et 3, y compris les bornes.

M est ainsi le cation trivalent correspondant à un élément de nature métallique.

Parmi les anions Z⁻, on peut citer aussi les imidures de formule (III) : 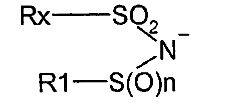 où R1 est un fluor, ou radical organique (c'est-à-dire contenant du carbone), contenant avantageusement au plus 30 atomes de carbone quand il n'est pas polymérique (c'est-à-dire ne constitue pas un lien de rattachement à un polymère) et où n est 1 ou 2, avantageusement 2, surtout si R1 est fluor.

En particulier Z- peut constituer un composé de formule (IV) : 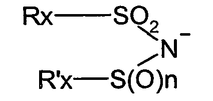 ou de formule (V) :

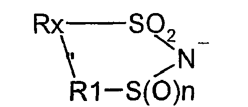

Dans le cas où Rₓ et R1 sont reliés, Rₓ et R1 doivent être divalents. Il s'ensuit, mais cela va sans dire, que dans ce cas R1 ne peut être fluor.

De très bons résultats sont obtenus lorsque q est égal à 3. Le carbone halogéné est avantageusement un carbone perfluoré et, plus préférentiellement, un groupe perfluorométhylène comme cela apparaîtra par la suite. L'autre anion ou les autres anions sont des anions organiques ou minéraux, de préférence des monoanions.

On peut citer parmi ces anions les sulfonates, les sulfates de monoalcoyle (quand ces derniers sont stables dans le milieu), les carboxylates, les halogénures, les halogénates (lorsque ces derniers ne sont pas trop oxydants pour le milieu), les phosphates, les phosphonates et les phosphinates, de préférence rendus monovalents par estérification partielle lorsque cela est nécessaire ; les pyrophosphates peuvent être envisagés dans les milieux où ils sont stables.

Il est toutefois préférable que ces anions autres que les sulfonates perhalogénés ne soient pas chélatants.

Il est également préférable que le pKₐ de l'acide associé à ces anions soit au plus égal à environ 10, de préférence à environ 5, plus préférentiellement à environ 2.

Il est préférable que l'acide associé ne soit pas plus acide que les acides halohydriques.

Ces sels jouant le rôle de catalyseurs peuvent être introduits déjà formés ou être réalisés in situ par neutralisation d'un oxyde de l'élément M, par mélange de sels de l'élément M, ou déplacement d'un acide plus faible ou plus volatil par ledit super acide ZH. Toutefois les faits in situ donnent en général des résultats inférieurs au sel préalablement synthétisé.

Selon la présente invention, la réaction de formation d'uréthane et notamment de polyuréthane peut être une réaction de transcarbamatation. Ce terme de transcarbamatation doit être pris lato sensu, c'est-à-dire qu'elle vise aussi le passage d'un pseudocarbamate (produit de réaction d'un isocyanate avec un dérivé hydroxylé ne constituant pas un alcool, par exemple choisi parmi les oximes ou les hydroxyamides) en uréthane ou polyuréthane par condensation avec un alcool ou plus généralement avec un polyol connu dans le domaine.

Ces catalyseurs sont avantageusement utilisés en quantité au moins égale à 0,5‰ et au plus égale à 5% en équivalent de fonction carbamate, de préférence entre 1‰ et 2%.

La réaction de transcarbamatation dépend des alcools et des carbamates utilisés. Elle est toutefois comprise entre 100 et 200°C, de préférence entre 120 et 180°C. Les carbamates utilisés sont ceux issus de la réaction d'une fonction isocyanate avec une fonction hydroxyle. Parmi les fonctions hydroxyles, il convient de citer les fonctions alcools, surtout celles des alcools volatiles à la température de réaction (température d'ébullition sous pression atmosphérique) et plus particulièrement le méthanol.

Parmi les autres fonctions hydroxyles que l'on peut citer comme présentant un avantage particulier, les fonctions phénols, les fonctions hydroxyles greffées sur un atome d'azote comme les hydroxy-imides, les oximes.

Les alcools qui se substituent à ces dérivés hydroxylés sont avantageusement des polyols (surtout di- et/ou triols), avantageusement primaires.

Les masses moléculaires peuvent varier dans un large domaine selon la forme du revêtement utilisé. Les masses moléculaires sont relativement élevées pouvant aller environ jusqu'à 20.000 lorsque l'on met en oeuvre les catalyseurs selon la présente invention dans une peinture poudre. En revanche, pour les applications les plus classiques, les polyols dépassent rarement une masse moléculaire de 3.000 environ.

Les masses moléculaires auxquelles il est fait référence sont des masses moléculaires moyenne en nombre Mₙ et sont définies par la technique de perméation de gel connue de "l'homme de métier". Plus spécifiquement, la masse moléculaire est déterminée par chromatographie par perméation de gel (GPC). La technique utilise comme gels, deux gels de polystyrène (ultrastyragel^{®} à 10⁴ et 500 Ä), le THF comme solvant et le soufre comme standard.

Les isocyanates donnant naissance ou correspondant aux carbamates préférés sont au moins partiellement des isocyanates aliphatiques, c'est-à-dire que la fonction isocyanate considérée est reliée par l'azote au squelette de la molécule isocyanate par un atome de carbone d'hybridation sp³.

Il est en outre souhaitable que, dans la structure du ou des isocyanates monomères (c'est-à-dire les isocyanates, en général des di-isocyanates, qui constituent les précurseurs des polyisocyanates utilisés comme «réticulant», et dont les plus couramment utilisés sont l'hexaméthylène di-isocyanate et le composé désigné par le terme isophorone di-isocyanate, ou IPDI), la partie du squelette reliant deux fonctions isocyanates comporte au moins un enchaînement polyméthylène (CH₂)_{π} où π représente un entier de 2 à 10, avantageusement de 4 à 8. Cette préférence joue sur les performances mécaniques.

Quand il y a plusieurs enchaînements, ces derniers peuvent être semblables ou différents. En outre, il est souhaitable que, dans le monomère considéré, ces enchaînements polyméthylène soient libres en rotation et donc exocycliques, s'il y a un cycle. Lorsque l'on utilise des prépolymères, ou oligomères, issus de plus d'un monomère, il est souhaitable que la condition relative à cet enchaînement polyméthylène se retrouve dans l'un au moins de ces monomères.

Les polyisocyanates préférés sont ceux qui présentent au moins une fonction isocyanate aliphatique. En d'autres termes, au moins une fonction isocyanate masquée selon l'invention est reliée au squelette par l'intermédiaire d'un carbone de type sp³ portant avantageusement un atome d'hydrogène, de préférence deux. Il est souhaitable que ledit carbone de type sp³ soit lui-même porté par un carbone de type sp³ et avantageusement muni d'un, de préférence de deux atomes d'hydrogène, et ce pour éviter que la fonction isocyanate considérée soit en position néopentylique.

En d'autres termes, il est conseillé de choisir comme monomère (lesquels sont en général porteurs de deux fonctions isocyanates) au moins un composé qui porte au moins une fonction isocyanate aliphatique qui ne soit ni secondaire ou tertiaire, ni néopentylique.

En cas de mélange obtenu à partir de plusieurs (en général deux) types de monomères, il est préférable que celui ou ceux des monomères qui répondent aux conditions ci-dessus et/ou (avantageusement "et") à la condition sur la présence d'enchaînement polyméthylène (CH₂)_{π}, représentent au moins 1/3, avantageusement 1/2, de préférence 2/3, des fonctions isocyanates masquées. Ainsi au cours de l'étude selon la présente invention, il a été obtenu d'excellents résultats avec des mélanges comportant 2/3 d'HMDT ("trimère" d'hexaméthylène di-isocyanate) avec de l'IPDI ou de l'IPDT ("trimère" d'IPDI), les deux étant masqués selon l'invention (le nBDI, norbornane di-isocyanate, et son trimère sont similaires).

On préfère bien sûr le cas où la totalité des isocyanates sont aliphatiques, et même répondent aux critères ci-dessus.

Selon la présente invention, l'isocyanate masqué, pur ou en mélange, est issu d'un polyisocyanate, c'est-à-dire possédant au moins deux fonctions isocyanates, avantageusement plus de deux (possibilités de valeurs fractionnaires puisqu'il s'agit en général de mélange d'oligomères plus ou moins condensés), lequel est lui-même le plus souvent issu d'une précondensation ou d'une prépolymérisation de di-isocyanate unitaire (parfois qualifié dans la présente description de "monomère").

La masse moléculaire moyenne de ces prépolymères ou de ces précondensats est en général au plus égale à 2.000 (un chiffre significatif), plus couramment à 1.000 (un chiffre significatif, de préférence deux).

Ainsi, parmi les polyisocyanates utilisés pour l'invention, on peut citer ceux du type biuret et ceux dont la réaction de di- ou trimérisation a conduit à des cycles à quatre, cinq ou six chaînons. Parmi les cycles à six, on peut citer les cycles isocyanuriques issus d'une homo- ou d'une hétérotrimérisation de divers di-isocyanates seuls, avec d'autre(s) isocyanate(s) [mono-, di-, ou polyisocyanate(s)] ou avec du gaz carbonique (ou bioxyde de carbone) ; dans ce cas on remplace un azote du cycle isocyanurique par un oxygène. Les oligomères à cycles isocyanuriques sont préférés.

Parmi les monomères les plus intéressants, il convient de citer ceux qui présentent un enchaînement polyméthylène tel que défini plus haut, exocyclique, évidemment y compris non cyclique. Parmi les monomères présentant un enchaînement polyméthylène on peut citer le tétraméthylène di-isocyanate éventuellement substitué par un groupe alcoyle avantageusement d'au plus quatre atomes de carbone, de préférence d'au plus deux atomes de carbone ; le pentaméthylène di-isocyanate éventuellement substitué par un groupe alcoyle, avantageusement d'au plus quatre atomes de carbone, de préférence d'au plus deux atomes de carbone et l'hexaméthylène di-isocyanate. Comme monomères de nature cycloaliphatique qui sont de préférence utilisés en association avec des isocyanates ayant des enchaînements polyméthylènes exocycliques, ou non cycliques, on peut citer les monomères et les composés issus des monomères suivants :
→ Les composés correspondant à l'hydrogénation du ou des noyaux aromatiques porteurs des fonctions isocyanates de monomères d'isocyanates aromatiques, et notamment du TDI (toluènediisocyanate) et des diisocyanates diphényles, le composé connu sous le sigle H₁₂MDl et les divers BIC [bis(isocyanatométhyl cyclohexane)] ; et surtout
→ le norbornane di-isocyanate souvent appelé par son sigle NBDl;
→ l'isophorone di-isocyanate ou IPDI ou 3-isocyanatométhyl-3,5-triméthyl-cyclohexylisocyanate.

Il convient de signaler que les catalyseurs selon la présente invention sont également favorables lorsque la réaction de formation d'uréthane comporte une réaction de passage d'une fonction urée à une fonction carbamate. La fonction urée (>N-CO-N<) doit être prise au sens large et vise surtout les composés qui constituent des isocyanates masqués par des dérivés, ou plus exactement des agents de masquage, porteurs d'un hydrogène réactif sur un azote. Ces isocyanates masqués permettent de conduire à des polyuréthanes au moyen d'alcools et notamment de polyols.

La notion d'agent de masquage correspond à celle qui a fait l'objet d'ouvrages tels que celui de Petersen et celui de Wicks.

La présente invention est particulièrement utile pour les isocyanates masqués dont la température de libération telle que définie dans le test à l'octanol (voir infra) est relativement élevée, c'est-à-dire au moins égale à 100°C, avantageusement à 120°C, de préférence à 130°C.

Les agents de masquage donnant les meilleurs résultats sont les agents de masquage présentant une fonction NH. Ainsi, avantageusement, l'agent de masquage, ou au moins l'un d'entre eux lorsqu'il y en a plus d'un, est choisi parmi les composés présentant une fonction >NH, avantageusement parmi les amines secondaires, les cycles azotés aromatiques à cinq chaînons, présentant de préférence au moins deux azotes.

Toutefois, ceux qui sont choisis parmi les composés porteurs de fonctions hydroxylées, avantageusement choisis parmi les alcools saturés de point d'ébullition inférieur à 150°C (avantageusement à 100°C), les phénols, les oximes et l'hydroxyamide.

Selon la présente invention, il est souhaitable que le métal correspondant au cation M ne présente pas de valence inférieure à 3.

Il est également avantageux que le métal soit un métal relativement lourd, c'est-à-dire que ledit métal appartient à une période supérieure à la quatrième période, avantageusement à la cinquième période. On peut se référer à la classification des périodiques qui faisait l'objet du supplément au bulletin de la Société Chimique de France n°1, en janvier 1966.

Les cations visés par la présente invention sont essentiellement ceux des terres rares (scandium, yttrium, lanthane et lanthanide) et les éléments de la classification périodique des éléments choisis parmi le gallium, l'arsenic, l'indium, l'étain, l'antimoine, le thallium et le bismuth.

Parmi les métaux donnant les meilleurs résultats, il convient de citer les lanthanides (lanthanide étant pris ici stricto sensu, c'est-à-dire qu'il ne comporte pas le lanthane) notamment l'yttrium et surtout le bismuth.

En ce qui concerne les sulfonates, il est souhaitable que Rₓ soit de formule:

GEA-(CX₂)ₚ-

où :
- les X, semblables ou différents, représentent un chlore, un fluor ou un radical de formule CₙF₂ₙ₊₁, avec n entier au plus égal à 5, de préférence à 2, avec la condition qu'au moins un des X soit fluor, fluor avantageusement porté par le carbone relié au soufre ;
- p représente un entier au plus égal à 2 ;
- GEA représente un groupe électro-attracteur (c'est-à-dire sigma p supérieur à zéro, avantageusement à 0,1, de préférence à 0,2) dont les éventuelles fonctions sont inertes dans les conditions de la réaction, avantageusement fluor ou un reste perfluoré de formule CₙF₂ₙ₊₁, avec n entier au plus égal à 8, avantageusement à 5, le nombre total de carbone de Rf étant avantageusement compris entre 1 et 15, de préférence entre 1 et 10.

Comme cela a été indiqué, il est préféré que le carbone portant la fonction sulfonate soit un carbone perfluoré et notamment un carbone constituant un perfluorométhylène (-CF₂-). Pour des raisons de facilité d'obtention industrielle, les premiers membres des groupes R_{f} (perfluoroalcoyle) sont préférés et notamment les acides trifluorométhanesulfonique (acide triflique correspondant au triflate) et les acides pentafluoroéthanesulfoniques donnent de bons résultats.

La présente invention a également pour objet des compositions d'isocyanates masqués, avantageusement sous la forme de carbamates (lato sensu, c'est-à-dire les fonctions correspondant à la séquence -N(R)- CO-O-, où R est un radical hydrocarboné, en général alcoyle, voire aryle, ou plus fréquemment un hydrogène) et comportant en outre un catalyseur de formule générale (I) suivante :

MY_{3-q}[(Rₓ)-SO₂-O-]_{q},

où:
- M représente un métal trivalent, de préférence connu, pour former des acides de Lewis ;
- Y est un anion ou une fonction anionique monovalente et ;
- Rₓ est un radical dont le carbone porteur de la fonction sulfonique est perhalogéné, et
- q est un entier choisi avantageusement entre 1 et 3, y compris les bornes.

La présente invention vise également un procédé de transcarbamatation où l'on utilise comme catalyseur de transcarbamatation les composés de formule :

MY _{3-q}[(Rₓ)-SO₂-O-]_{q},

où :
- M représente un métal trivalent, de préférence connu pour former des acides de Lewis ;
- Y est un anion ou une fonction anionique monovalente et ;
- Rₓ est un radical dont le carbone porteur de la fonction sulfonique est perhalogéné, et
- q est un entier choisi avantageusement entre 1 et 3, y compris les bornes.

Ces catalyseurs permettent de réaliser la réaction à des températures inférieures à 200°C, le plus souvent inférieures à 180°C.

En général pour obtenir une cinétique suffisante, il convient de se placer à une température au moins égale à 100°C, de préférence à une température au moins égale à 120°C.

### Test à l'octanol

- Température de "libération" :: C'est la température la plus faible à laquelle (ou de "déblocage") l'agent de masquage de l'isocyanate masqué est déplacé à hauteur de 9/10 (arrondi mathématique) par un monoalcool primaire (l'alcool primaire est en général l'octanol).
- Durée de vie au stockage:: Pour s'assurer une bonne durée de vie au stockage, il est préférable de choisir des fonctions isocyanates masquées dont le test à l'octanol montre une "libération" à 80°C, avantageusement à 90°C, au plus égale à 90%.
- Avancement de la réaction:: On considère que la réaction est complète si elle est réalisée à plus de 90%.

Les exemples non limitatifs suivants illustrent l'invention. Ces exemples, afin d'éviter tous problèmes d'interaction, ont sensiblement porté sur des isocyanates monofonctionnels. On a utilisé l'isocyanate de n-hexyle.

### Exemple 1

### Mode opératoire général :

### Choix du modèle réactionnel

Le choix s'est porté sur l'utilisation d'isocyanates aliphatiques qui sont plus coûteux mais qui donnent accès à des polyuréthanes présentant :
- une meilleure résistance à l'U.V.,
- une couleur plus stable,
- une excellente tenue en usage externe.

L'action d'alcools primaires donne des carbamates très stables thermiquement par rapport aux isocyanates aromatiques.

Le méthanol a été choisi en tant qu'agent bloquant de l'isocyanate d'alcoyle. En effet, cet alcool présente un coût peu élevé, une masse moléculaire faible, ainsi qu'une toxicité limitée.

Un tel agent bloquant favorise une limitation des composés organiques volatiles (C.O.V.) par rapport aux autres agents. Cette méthodologie s'inscrit bien dans le cadre du développement d'une chimie plus propre, axe privilégié de la recherche contemporaine.

### Réactifs mis en jeu

L'isocyanate d'alcoyle choisi est l'isocyanate d'hexyle. Son blocage est réalisé très simplement par le méthanol sous chauffage classique pendant 4 heures. La réaction conduit à la formation du N-hexylméthyluréthane qui servira de réactif de départ au cours de la réaction de transcarbamatation.

*n*-hex-N=C=O + MeOH → n-hex-NH-CO-OMe

L'alcool primaire choisi pour réaliser la transcarbamatation est l'octan-1-ol. Son action sur l'isocyanate d'hexyle sous chauffage classique pendant 4 heures conduit à la formation du produit d'arrivée : le N-hexyloctyluréthane.

*n*-hex-N=C=O + n-OctOH → n-hex-NH-CO-O-n-Oct

La caractérisation de ce produit par les techniques d'analyse classique a été nécessaire pour réaliser un suivi cinétique de la réaction de transcarbamatation par chromatographie en phase gazeuse (temps de rétention et coefficient de réponse).

### Etude cinétique

La réaction de transcarbamatation étudiée conduite en l'absence de solvant et en présence de 1 % molaire de catalyseur (pour avoir une réaction rapide) est schématisée ci-dessous : 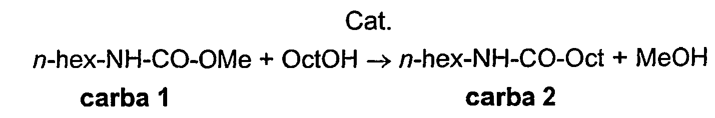 *n*-hex-NH-CO-OMe + OctOH → *n*-hex-NH-CO-Oct + MeOH **carba 1 carba 2**
- Carba 1 : 3,14 mmoles (500 mg)
- OctOH : 1 éq. ou 10 éq.
- Référence interne (hexadécane) : 0,5 éq.
- Cat. : 1% mol.
- 130°C

### Résultats cinétiques de différents catalyseurs testés

| **Catalyseur** | **k en mol⁻¹.L. min⁻¹** | **Rdt (%)** |
|---|---|---|
| Bu₂Sn(laurate)₂ référence | 1,10.10⁻³ | 26 |
| BiPh₃ comparatif | 0 | 0 |
| BiCl₃, comparatif | 3,80.10⁻⁵ | 2 |
| **Bi(OTf)₃** | **5,54.10⁻³** | **70** |
| Yb(OTf)₃ | 8,76.10⁻⁴ | 30 |
| Sm(OTf)₃ | **2,0.10⁻³** | **45** |

Le triphénylbismuth et le chlorure de bismuth affichent une activité catalytique quasiment nulle. Quant au triflate de bismuth, il fait preuve d'une efficacité remarquable avec une constante de vitesse de 4,54.10⁻³ mol⁻¹.L.min⁻¹ et un rendement final de 70%, résultat tout à fait comparable au distannoxane dibromé.

### Essais réalisé avec des hexaméthylènedicarbamate de méthyle

### Exemple 2 - Synthèse du carbamate d'octyle et de méthyle

On charge dans un réacteur 41, 44 g de 1-n-octyle isocyanate et 17 g de méthanol. On agite à 60°C pendant 8 heures. Le produit est ensuite refroidi à température ambiante. Il cristallise. On filtre le produit cristallisé (38 g). Le produit cristallisé est lavé par de l'hexane froid pour donner 19 g de produit pur. Le carbamate est caractérisé par analyse RMH du proton et infra rouge.

### Exemple 3 - Synthèse du carbamate d'octyle et d'octyle

On opère de la même manière que pour l'exemple 1, à partir de 1,7 g de 1-n-octyle isocyanate (1 équivalent molaire) et 2,86 g de n-octanol (2 équivalents molaires).

Après 4 heures, on contrôle par analyse infra rouge l'absence de bandes isocyanates. On purifie le produit comme décrit précédemment.

Ce produit sert de référence.

Le carbamate est caractérisé par analyse RMH du proton et infra rouge.

### Exemple 4 - Synthèse du carbamate d'hexyle et d'octale

On procède comme pour l'exemple 1, sauf que l'on utilise le n-1-hexyl isocyanate (5 g) et l'octanol (10 g).

On récupère environ 5 g de produit.

Le carbamate est caractérisé par analyse RMH du proton et infra rouge.

Ce produit sert de référence.

### Exemple 5 - Synthèse du carbamate d'hexyle et de méthyle

On procède comme pour l'exemple 1, sauf que l'on utilise le n-1-hexyl isocyanate (5 g) et le méthanol (1,8 g).

On récupère environ 5,7 g de produit.

Le carbamate est caractérisé par analyse RMH du proton et infra rouge.

### Exemple 6 - Conditions d'analyses de la réaction

La réaction de transcarbamatation est suivie par analyse chromatographique en phase gaz en utilisant une colonne.

On élue les composés par un gradient de température 100°C à 220°C avec une augmentation de 10°C/mn. La température d'injection est de 100°C.

| Composé | Temps d'élution |
|---|---|
| octanol | 5,22 |
| trichlorobenzène (référence interne) | 7,41 |
| carbamate d'hexyle et de méthyle | 8,32 |
| carbamate d'octyle et de méthyle | 11,14 |
| carbamate d'hexyle et d'octyle | 16,41 |
| carbamate d'octyle et d'octyle | 21 |

### Exemple 7 - Synthèse du dicarbamate de méthyle et d'hexaméthylène

On procède comme pour l'exemple 1. On utilise 171 g d'examéthylène di-isocyanate et 271 g de méthanol.

On contrôle le titre NCO par mesure du titre par réaction avec la dibutylamine en excès et dosage de la dibutylamine en excès par HCL (dosage classique des isocyanates).

Le dicarbamate de méthyle cristallise à froid. Il est lavé à l'hexane puis séché. L'analyse RMN 1H donne NH 4,7 ppm ; -CH3O 3,6 ppm ; -CH2N 3,1 ppm ; -(CH2)4 1,4 et 1,27 ppm .

### Mode opératoire général des tests de catalyses de transcarbamatation

On travaille à 160°C dans un réacteur agité, avec 1 équivalent molaire de carbamate de méthyle et 1 équivalent molaire de octanol 1. On ajoute 1% molaire de catalyseur.

### Exemple 8 - Réaction de transcarbamatation catalysé par des triflates métalliques à 160°C

| Carbamate de méthyle de départ utilisé | Catalyseur utilisé / température / 4 heures de réaction | Taux de transformation du carbamate de méthyle en octyle |
|---|---|---|
| Carbamate octyle et méthyle | La (Triflate)3 / 160°C | 62% |
| Carbamate octyle et méthyle | Yb (Triflate)3 / 160°C | 99% |

### Exemple 9 (comparatif) - Réaction de transcarbamatation catalysé par des triflimides métalliques à 160°C

| Carbamate de méthyle de départ utilisé | Catalyseur utilisé / température / 4 heures de réaction | Taux de transformation du carbamate de méthyle en octyle |
|---|---|---|
| Carbamate octyle et méthyle | Bis (trifluorométhane sulfonimide de potassium)/160°C | 42% |
| Carbamate octyle et méthyle | Bis (trifluorométhane sulfonimide de lithium)/160°C | 51% |
| Carbamate octyle et méthyle | Bis (trifluorométhane sulfonimide de magnésium)/160°C | 45% |

Les mélanges de catalyseurs préparés in situ se révèlent être moins efficaces que les dérivés préparés avant la réaction.

### Exemple 10 - Réaction de transcarbamatation catalysée par des mélanges de sels métalliques à 160°C

| Carbamate de méthyle de départ utilisé | Catalyseur utilisé / température / 4 heures de réaction | Taux de transformation du carbamate de méthyle en octyle |
|---|---|---|
| Carbamate octyle et méthyle | 1 eq molaire acide triflique + 1 équivalent molaire BiCl3/130°C | 33% |
| Carbamate octyle et méthyle | 2eq molaire acide triflique + 1 équivalent molaire BiCl3/130°C | 29% |
| Carbamate octyle et méthyle | 3 eq molaire acide triflique + 1 équivalent molaire BiCl3/130°C | 34% |
| Carbamate octyle et méthyle | 1 eq molaire bis (trifluorométhanes ulfon) imide (TFSIH) + 1 équivalent molaire Bil3 / 130°C | 28% |
| Carbamate octyle et méthyle | 2 eq molaire TFSIH + 1 équivalent molaire BiCl3/130°C | 20% |
| Carbamate octyle et méthyle | 3 eq molaire TFSIH + 1 équivalent molaire BiCl3/130°C | 25% |
| Carbamate octyle et méthyle | 1 eq molaire acide triflique + Bi2O5 1 équivalent molaire/130°C | 39% |

### Exemple 11 - Réaction de transcarbamatation de dicarbamate de méthyle et d'hexaméthylène et composés diols ou polyols diol

On procède à la réaction en présence des meilleurs catalyseurs obtenus. On vérifie si on obtient un gel. On mesure le taux de transformation du dicarbamate de méthyle et d'hexaméthylène par extraction avec un solvant polaire la N-méthylpyrolidone et dosage par CPG. On montre que dans tous les cas la quantité de carbamate de diméthyle et d'hexaméthylène est inférieure à 10%.

Ces exemples montrent que l'on peut obtenir des polyuréthannes par réaction de transcarbamatation catalysée par les dérivés organométalliques de l'invention.

| Carbamate de méthyle de départ utilisé/polyol ratio NCO/OH = 1 | Catalyseur utilisé / température/ 4 heures de réaction | Résultat qualitatif |
|---|---|---|
| Dicarbamate de méthyle et d'hexaméthylène/hexane diol 1,6 | Yb (triflate)3/160°C | Obtention d'un gel |
| Dicarbamate de méthyle et d'hexaméthylène/hexane diol 1,6 | Bi (triflate)3/160°C | Obtention d'un gel |
| Dicarbamate de méthyle et d'hexaméthylène/hexane diol 1,6 | Bi (triflate)3/130°C | Obtention d'un gel |
| Dicarbamate de méthyle et d'hexaméthylène/polyéthylène glycol masse 1000 | Yb (triflate)3/160°C | Obtention d'un gel |

### Exemples comparatifs 12 - Réaction de transcarbamatation catalysée par des acides protiques

On travaille à 160°C dans un réacteur agité, avec 1 équivalent molaire de carbamate de méthyle et 1 équivalent molaire d'octanol 1. On ajoute 1 % molaire de catalyseur acide. On laisse en milieu fermé sous agitation.

Les résultats sont analysés après 6 heures à 160°C et 130°C par chromatographie phase gaz.

Les taux de transcarbamatation mesurés sont faibles par rapport à ceux mesurés avec les acides de Lewis.

| Carbamate de méthyle de départ utilisé | Catalyseur utilisé / température | % molaire de carbamate d'octyle mesuré |
|---|---|---|
| Carbamate octyle et méthyle | Acide triflique/160°C | 8% Carbamate octyle - octyle |
| Carbamate octyle et méthyle | Bis (trifluorométhane sulfon)imide/160°C | 5,6% Carbamate octyle et octyle |
| Carbamate hexyle et méthyle | Acide triflique/160°C | 10% Carbamate octyle - octyle |
| Carbamate hexyle et méthyle | Bis (trifluorométhane sulfon)imide/160°C | 6% Carbamate octyle et octyle |

## Revendications

1. Utilisation comme catalyseur pour la formation d'urethane, par réaction de transcarbamtation, de composés répondant à la formule (I) générale suivante :
MY_{3-q}[Z]_{q},
où:
• Z est le radical correspondant à l'anion d'un super acide dont la constante de Hammett est au moins égale, avantageusement supérieure à 13
• M représentant un métal trivalent, de préférence connu pour former des acides de Lewis ;
• Y est un anion ou une fonction anionique monovalente et ;
• q est un entier choisi avantageusement entre 1 et 3, y compris les bornes.

2. Utilisation selon la revendication 1, **caractérisée par le fait que** Z est de formule :
(Rₓ)-SO₂-□-
où:
• a fonction sulfonique est perhalogéné, et
• □ est un oxygène ou un atome de la colonne V, de préférence l'azote, monosubstitué par un radical électro-attractteur, avantageusement par un radical de formule (R'ₓ)-SO₂-, où R'ₓ est choisi parmi les mêmes radicaux que Rₓ.

3. Utilisation selon les revendications 1 et 2, **caractérisée par le fait que** □ est un oxygène.

4. Utilisation selon les revendications 1 à 3, **caractérisée par le fait que** la réaction de formation d'uréthane comporte une réaction de passage d'une fonction urée (>N-CO-N<) à une fonction carbamate (>N-CO-O-).

5. Utilisation selon les revendications 1 à 4, **caractérisée par le fait que** le précurseur de la fonction uréthane est une fonction isocyanate masquée.

6. Utilisation selon la revendication 5, **caractérisée par le fait que** l'agent de masquage, ou au moins l'un d'entre eux lorsqu'il y en a plus d'un, est choisi parmi les composés porteurs de fonction hydroxylée avantageusement choisi parmi les alcools saturés de point d'ébullition inférieur à 150°C (avantageusement à 100°C), les phénols, les oximes et l'hydroxyamide.

7. Utilisation selon les revendications 5 et 6 **caractérisée par le fait que** l'agent de masquage, ou au moins l'un d'entre eux lorsqu'il y en a plus d'un, est choisi parmi les composés présentant une fonction >NH, avantageusement parmi les amines secondaires, les cycles azotés aromatiques à cinq chaînons, présentant de préférence au moins deux azotes.

8. Utilisation selon les revendications 1 à 7, **caractérisée par le fait que** ledit métal est un métal ne présentant pas de valence inférieure à 3.

9. Utilisation selon les revendications 1 à 8, **caractérisée par le fait que** ledit métal appartient à une période supérieure à la quatrième, avantageusement à la cinquième période.

10. Utilisation selon les revendications 1 à 9, **caractérisée par le fait que** ledit métal est un lanthanide ou le bismuth.

11. Utilisation selon les revendications 2 à 10, **caractérisée par le fait que** Rₓ est de formule :
GEA-(CX₂)ₚ-
ou :
• les X, identiques ou différents, représentent un chlore, un fluor ou un radical de formule CₙF₂ₙ₊₁ avec n entier au plus égal à 5, de préférence à 2, avec la condition qu'au moins un des X soit fluor, fluor avantageusement porté par le carbone relié au soufre ;
• p représente un entier au plus égal à 2 ;
• GEA représente un groupe électro-attracteur (c'est-à-dire sigma p supérieur à zéro, avantageusement à 0,1, de préférence à 0,2) dont les éventuelles fonctions sont inertes dans les conditions de la réaction, avantageusement fluor ou un reste perfluoré de formule CₙF₂ₙ₊₁ avec n entier au plus égal à 8, avantageusement à 5, le nombre total de carbone de Rf étant avantageusement compris entre 1 et 15, de préférence entre 1 et 10.

12. Composition **caractérisée par le fait qu'**elle comporte au moins un carbamate susceptible d'être obtenu par l'action d'un composé hydroxylé sur un isocyanate avantageusement aliphatique, et au moins un des composés répondant à la formule (I) générale suivante :
MY_{3-q}[Z-]_{q},
où:
• Z est le radical correspondant à l'anion d'un super acide dont la constante de Hammett est au moins égale, avantageusement supérieure à 13.
• M représentant un métal trivalent, de préférence connu pour former des acides de Lewis ;
• Y est un anion ou une fonction anionique monovalente et ;
• q est un entier choisi avantageusement entre 1 et 3, y compris les bornes.

13. Composition selon la revendication 14, **caractérisée par le fait qu'**elle comporte en outre un alcool ou un polyalcool avantageusement primaire.

14. Composition selon les revendications 12 et 13, **caractérisée par le fait que** ledit composé hydroxylé est choisi parmi les alcools volatils à une température au plus égale à 150°C et parmi les agents de masquage hydroxylés.

15. Composition selon les revendications 12 à 14, **caractérisée par le fait que** ledit composé hydroxylé est le méthanol.

16. Composition selon les revendications 12 à 15, **caractérisée par le fait que** ledit composé hydroxylé est choisi parmi les agents masquants, avantageusement parmi les phénols et les oximes.

17. Procédé de transcarbamatation, **caractérisé par le fait que** l'on utilise comme catalyseur de transcarbamatation les composés répondant à la formule (I) générale suivante :
MY_{3-q}[Z-]_{q},
où:
• Z est le radical correspondant à l'anion d'un super acide dont la constante de Hammett est au moins égale, avantageusement supérieure à 13.
• M représentant un métal trivalent, de préférence connu pour former des acides de Lewis ;
• Y est un anion ou une fonction anionique monovalente et ;
• q est un entier choisi avantageusement entre 1 et 3, y compris les bornes.

18. Procédé selon la revendication 17, **caractérisé par le fait que** la réaction de transcarbamatation est menée à une température comprise entre 100 et 200°C, avantageusement entre 120 et 180°C.

## Claims

1. Use as catalyst for the formation of urethane, by a transcarbamation reaction, of compounds corresponding to the following general formula (I):
MY_{3-q}[Z]_{q},
where:
• Z is the radical corresponding to the anion of a superacid for which the Hammett constant is at least equal to, advantageously greater than, 13;
• M represents a trivalent metal, preferably known for forming Lewis acids;
• Y is a monovalent anion or anionic functional group, and;
• q is an integer advantageously chosen between 1 and 3, including the limits.

2. Use according to Claim 1, **characterized in that** Z is of formula:
(Rₓ) -SO₂-ζ-
where:
• Rₓ is a fluorine or, advantageously, a radical, the carbon of which carrying the sulphonic functional group is perhalogenated, and
• ζ is an oxygen or an atom from Group V, preferably nitrogen, monosubstituted by an electron-withdrawing radical, advantageously by a radical of formula (R'ₓ)-SO₂-, where R'ₓ is chosen from the same radicals as Rₓ.

3. Use according to Claims 1 and 2, **characterized in that** ζ is an oxygen.

4. Use according to Claims 1 to 3, **characterized in that** the reaction for the formation of urethane comprises a reaction in which a urea functional group (>N-CO-N<) is converted to a carbamate functional group (>N-CO-O-) .

5. Use according to Claims 1 to 4, **characterized in that** the precursor of the urethane functional group is a masked isocyanate functional group.

6. Use according to Claim 5, **characterized in that** the masking agent, or at least one of them when there is more than one of them, is chosen from compounds carrying a hydroxylated functional group advantageously chosen from saturated alcohols with a boiling point below 150°C (advantageously below 100°C), phenols, oximes and hydroxyamides.

7. Use according to Claims 5 and 6, **characterized in that** the masking agent, or at least one of them when there is more than one of them, is chosen from compounds exhibiting an >NH functional group, advantageously from secondary amines or five-membered aromatic nitrogenous rings preferably exhibiting at least two nitrogens.

8. Use according to Claims 1 to 7, **characterized in that** said metal is a metal not exhibiting a valency of less than 3.

9. Use according to Claims 1 to 8, **characterized in that** said metal belongs to a period greater than the fourth, advantageously than the fifth, period.

10. Use according to Claims 1 to 9, **characterized in that** said metal is a lanthanide or bismuth.

11. Use according to Claims 2 to 10, **characterized in that** Rₓ is of formula:
EWG- ( CX₂ ) ₚ-
where:
• the X groups, which are identical or different, represent a chlorine, a fluorine or a radical of formula CₙF₂ₙ₊₁, with n an integer at most equal to 5, preferably to 2, with the condition that at least one of the X groups is fluorine, fluorine -advantageously carried by the carbon connected to the sulphur;
• p represents an integer at most equal to 2;
• EWG represents an electron-withdrawing group (that is to say, σₚ of greater than zero, advantageously than 0.1, preferably than 0.2), the possible functional groups of which are inert under the conditions of the reaction, advantageously fluorine or a perfluorinated residue of formula CₙF_{2n+1,} with n an integer at most equal to 8, advantageously to 5, the total carbon number of Rf advantageously being between 1 and 15, preferably between 1 and 10.

12. Composition, **characterized in that** it comprises at least one carbamate capable of being obtained by reaction of a hydroxylated compound with an isocyanate, advantageously an aliphatic isocyanate, and at least one of the compounds corresponding to the following general formula (I):
MY_{3-q}[Z]_{q},
where:
• Z is the radical corresponding to the anion of a superacid for which the Hammett constant is at least equal to, advantageously greater than, 13;
• M represents a trivalent metal, preferably known for forming Lewis acids;
• Y is a monovalent anion or anionic functional group, and;
• q is an integer advantageously chosen between 1 and 3, including the limits.

13. Composition according to Claim 14, **characterized in that** it additionally comprises an alcohol or polyalcohol which is advantageously primary.

14. Composition according to Claims 12 and 13, **characterized in that** said hydroxylated compound is chosen from alcohols which are volatile at a temperature at most equal to 150°C and from hydroxylated masking agents.

15. Composition according to Claims 12 to 14, **characterized in that** said hydroxylated compound is methanol.

16. Composition according to Claims 12 to 15, **characterized in that** said hydroxylated compound is chosen from masking agents, advantageously from phenols and oximes.

17. Transcarbamation process, **characterized in that** use is made, as transcarbamation catalyst, of the compounds corresponding to the following general formula (I):
MY_{3-q}[Z]_{q},
where:
• Z is the radical corresponding to the anion of a superacid for which the Hammett constant is at least equal to, advantageously greater than, 13;
• M represents a trivalent metal, preferably known for forming Lewis acids;
• Y is a monovalent anion or anionic functional group, and;
• q is an integer advantageously chosen between 1 and 3, including the limits.

18. Process according to Claim 17, **characterized in that** the transcarbamation reaction is carried out at a temperature of between 100 and 200°C, advantageously between 120 and 180°C.

## Patentansprüche

1. Verwendung von Verbindungen der folgenden allgemeinen Formel (I) :
MY_{3-q} [Z]_{q}
worin:
• Z für den dem Anion einer Supersäure mit einer Hammett-Konstante von mindestens 13, vorteilhafterweise mehr als 13, entsprechenden Rest steht,
• M für ein dreiwertiges Metall, das vorzugsweise für die Bildung von Lewis-Säuren bekannt ist, steht;
• Y für ein Anion oder eine einwertige anionische Funktion steht und
• q für eine ganze Zahl steht, die vorteilhafterweise zwischen 1 und 3 einschließlich der Grenzen ausgewählt ist;
als Katalysator zur Bildung von Urethan durch Umcarbamylierungsreaktion.

2. Verwendung nach Anspruch 1, **dadurch gekennzeichnet, daß** Z die folgende Formel hat:
(Rₓ) -SO₂-□-
worin:
• Rₓ für Fluor oder vorteilhafterweise einen Rest, in dem der die Sulfonylfunktion tragende Kohlenstoff perhalogeniert ist, steht und
• □ für Sauerstoff oder ein durch einen elektronenanziehenden Rest, vorteilhafterweise einen Rest der Formel (R'ₓ)-SO₂ worin R'ₓ unter den gleichen Resten wie Rₓ ausgewählt ist, substituiertes Atom der Gruppe V, vorzugsweise Stickstoff, steht.

3. Verwendung nach den Ansprüchen 1 und 2, **dadurch gekennzeichnet, daß** □ für Sauerstoff steht.

4. Verwendung nach den Ansprüchen 1 bis 3, **dadurch gekennzeichnet, daß** die Urethanbildungsreaktion eine Reaktion umfaßt, bei der eine Harnstoffunktion (>N-CO-N<) in eine Carbamatfunktion (>N-CO-O-) umgewandelt wird.

5. Verwendung nach den Ansprüchen 1 bis 4, **dadurch gekennzeichnet, daß** es sich bei dem Vorläufer der Urethanfunktion um eine maskierte Isocyanatfunktion handelt.

6. Verwendung nach Anspruch 5, **dadurch gekennzeichnet, daß** das Maskierungsmittel oder mindestens ein Maskierungsmittel bei Vorliegen mehrerer Maskierungsmittel unter Verbindungen mit einer Hydroxylfunktion, vorteilhafterweise ausgewählt unter gesättigten Alkoholen mit einem Siedepunkt von weniger als 150°C (vorteilhafterweise unter 100°C), Phenolen, Oximen und Hydroxyamid, ausgewählt ist.

7. Verwendung nach den Ansprüchen 5 und 6, **dadurch gekennzeichnet, daß** das Maskierungsmittel oder mindestens ein Maskierungsmittel bei Vorliegen mehrerer Maskierungsmittel unter Verbindungen mit einer >NH-Funktion und vorteilhafterweise unter sekundären Aminen, fünfgliedrigen aromatischen stickstoffhaltigen Ringen, die vorzugsweise mindestens zwei Stickstoffatome aufweisen, ausgewählt ist.

8. Verwendung nach den Ansprüchen 1 bis 7, **dadurch gekennzeichnet, daß** es sich bei dem Metall um ein Metall handelt, das keine Wertigkeit von weniger als 3 aufweist.

9. Verwendung nach den Ansprüchen 1 bis 8, **dadurch gekennzeichnet, daß** das Metall zu einer Periode gehört, die höher als die vierte und vorteilhafterweise höher als die fünfte Periode ist.

10. Verwendung nach den Ansprüchen 1 bis 9, **dadurch gekennzeichnet, daß** es sich bei dem Metall um ein Lanthanid oder Bismut handelt.

11. Verwendung nach den Ansprüchen 2 bis 10, **dadurch gekennzeichnet, daß** Rₓ die folgende Formel hat:
EAG- (CX₂) p-
worin:
• die Gruppen X gleich oder verschieden sind und für Chlor, Fluor oder einen Rest der Formel CₙF₂ₙ₊₁, worin n eine ganze Zahl von höchstens 5 und vorteilhafterweise höchstens 2 bedeutet, stehen, mit der Maßgabe, daß mindestens eine der Gruppen X für Fluor steht, welches vorteilhafterweise an den an den Schwefel gebundenen Kohlenstoff gebunden ist;
• p für eine ganze Zahl von höchstens 2 steht;
• EAG für eine elektronenanziehende Gruppe (d.h. sigma p größer als null, vorteilhafterweise größer als 0,1, vorzugsweise größer als 0,2), deren eventuelle Funktionen unter den Reaktionsbedingungen inert sind, vorteilhafterweise Fluor oder einen perfluorierten Rest der Formel CₙF₂ₙ₊₁, worin n eine ganze Zahl von höchstens 8 und vorteilhafterweise höchstens 5 bedeutet, wobei die Gesamtkohlenstoffzahl von Rf vorteilhafterweise zwischen 1 und 15 und vorzugsweise zwischen 1 und 10 liegt, steht.

12. Zusammensetzung, **dadurch gekennzeichnet, daß** sie mindestens ein Carbamat enthält, das durch Reaktion einer Hydroxylverbindung mit einem vorteilhafterweise aliphatischen Isocyanat und mindestens einer der Verbindungen der folgenden allgemeinen Formel (I):
MY_{3-q} [Z] q
worin:
• Z für den dem Anion einer Supersäure mit einer Hammett-Konstante von mindestens 13, vorteilhafterweise mehr als 13, entsprechenden Rest steht,
• M für ein dreiwertiges Metall, das vorzugsweise für die Bildung von Lewis-Säuren bekannt ist, steht;
• Y für ein Anion oder eine einwertige anionische Funktion steht und
• q für eine ganze Zahl steht, die vorteilhafterweise zwischen 1 und 3 einschließlich der Grenzen ausgewählt ist;
erhältlich ist.

13. Zusammensetzung nach Anspruch 12, **dadurch gekennzeichnet, daß** sie außerdem einen Alkohol oder Polyalkohol, der vorteilhafterweise primär ist, enthält.

14. Zusammensetzung nach den Ansprüchen 12 und 13, **dadurch gekennzeichnet, daß** die Hydroxylverbindung unter Alkoholen, die bei einer Temperatur von höchstens 150°C flüchtig sind, und unter hydroxylgruppenhaltigen Maskierungsmitteln ausgewählt ist.

15. Zusammensetzung nach den Ansprüchen 12 bis 14, **dadurch gekennzeichnet, daß** es sich bei der Hydroxylverbindung um Methanol handelt.

16. Zusammensetzung nach den Ansprüchen 12 bis 15, **dadurch gekennzeichnet, daß** die Hydroxylverbindung unter Maskierungsmitteln, vorteilhafterweise unter Phenolen und Oximen, ausgewählt ist.

17. Umcarbamylierungsverfahren, **dadurch gekennzeichnet, daß** man als Umcarbamylierungskatalysator die Verbindungen der folgenden allgemeinen Formel (I):
MY_{3-q} [Z]_{q}
worin:
• Z für den dem Anion einer Supersäure mit einer Hammett-Konstante von mindestens 13, vorteilhafterweise mehr als 13, entsprechenden Rest steht,
• M für ein dreiwertiges Metall, das vorzugsweise für die Bildung von Lewis-Säuren bekannt ist, steht;
• Y für ein Anion oder eine einwertige anionische Funktion steht und
• q für eine ganze Zahl steht, die vorteilhafterweise zwischen 1 und 3 einschließlich der Grenzen ausgewählt ist;
verwendet.

18. Verfahren nach Anspruch 17, **dadurch gekennzeichnet, daß** die Umcarbamylierungsreaktion bei einer Temperatur zwischen 100 und 200°C und vorteilhafterweise zwischen 120 und 180°C durchgeführt wird.
